# EUROPEAN PATENT APPLICATION

(11) **EP 1 739 184 A1**
(43) Date of publication of application: **03.01.2007**
(21) Application number: 05425463.6
(22) Date of filing: 28.06.2005
(51) Int. Cl.: C12P 17/02

(54) **Method for the production of taxol and/or taxanes from cultures of hazel cells**

(71) Applicant: Centro Biotecnologie Avanzate, 16132 Genova (IT)
(72) Inventor: Miele, Mariangela, 16132 Genova (IT); Armirotti, Andrea, 16132 Genova (IT); Balbi, Alessandro, 16132 Genova (IT); Bestoso, Federica, 16132 Genova (IT); Damonte, Gianluca, 16132 Genova (IT); Mazzei, Mauro, 16132 Genova (IT)
(74) Representative: Ferreccio, Rinaldo

(57) **Abstract**

Method for the production of taxol and/or taxanes, comprising the steps of: a) inducing the formation of callus from a plant tissue explant, through *in vitro* culturing in a suitable nutritient medium, b) cultivating the callus in a liquid medium to obtain a cell suspension culture capable of producing taxol and/or taxanes, c) recovering the taxol and/or the taxanes from the cells and/or from the culture medium of the cell suspension obtained from the callus in which the tissue explant is obtained from a plant of the genus *Corylus,* in particular *Corylus avellana*.

## Description

### Field of application

The present invention refers in general to the field of the production of active ingredients through cell cultures.

In particular, the invention concerns a method for the production of taxol and taxanes from cultures of hazel cells.

### Prior art

It has been known for many decades that various types of taxus, in particular *Taxus Brevifolia,* contain a cytotoxic active ingredient, used as anti-tumour agent, known as taxol or paclitaxel. Paclitaxel is a complex tricyclic diterpenoid with a side amide group, of the following formula (Wani et al., 1971):

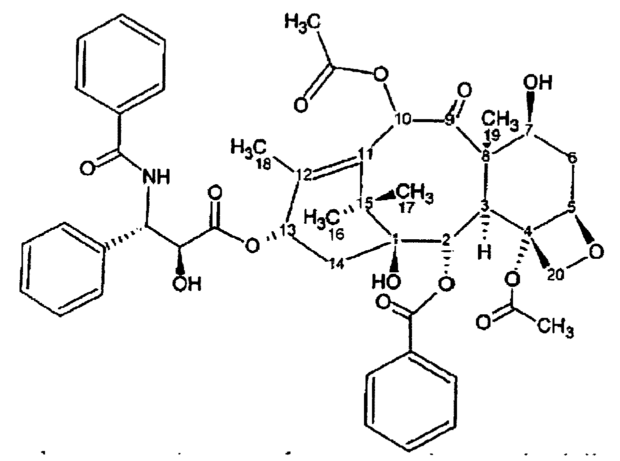

This molecule, approved as an anti-tumour drug by the FDA in 1992, is one of the most promising drugs in the treatment of various types of tumour, like for example ovarian carcinoma, breast carcinoma, Kaposi's sarcoma, tumour of the colon and of the lungs, melanoma, lymphoma and polycystic kidney disease (McGuire et al., 1989; Woo et al., 1994; Skell 1999).

Ever since taxol was approved for clinical use, substantial availability problems have arisen.

The species that contains the greatest amount of taxol is *Taxus brevifolia,* the most widespread taxus species in North America, and the bark is the comparatively richer part for active ingredient. North American taxuses were thus debarked for the production of the drug. The taxol yield is, however,rather low (0.01-0.03 %); indeed, to obtain 1 kg of active ingredient about 7 tons of bark are needed, which is the equivalent of 2000-3000 trees, and the debarking of taxus causes it to die.

Since taxus trees, although quite common in the North-Western forests of America, are hardly ever a dominant species andas they have very slow growth rates, their large-scale debarking risked putting the conservation of the species in serious danger. The search for alternative and renewable sources of taxol thus began.

Initially the possibility of producing taxol by total synthesis was explored but despite the efforts of many research laboratories, the best result obtained involved an overall yield of 2% and extremely high costs (Holton et al., 1994).

Therefore, attention was turned to the possibility of obtaining the molecule semi-synthetically, starting from its natural precursors. Currently, the main precursor used in the semi-synthesis of taxol is 10-deacetylbaccatin III, which can be extracted with a good yield (about 0.1%) from *T. baccata* leaves. The method for the semi-synthesis of taxol officially approved by the FDA was devised by Holton and co-workers (Holton et al., 1995) and involves four stages only starting from 10-deacetylbaccatin III, with a taxol yield of over 80%.

This method currently constitutes the only commercially viable way of producing taxol with a good yield, but it still suffers from some limitations, linked to the need to extract the precursor from the taxus plants.

Firstly, the taxane content is subject to considerable variations, both in quality and in quantity, due to numerous factors including the species, the *cultivar,* the age of the plant, the environmental conditions, the season, the soil, the temperature.

Moreover, the slow growth rate of taxus trees is a factor that limits the extraction of taxanes.

Finally, the precursor must necessarily undergo an expensive purification procedure.

In the light of the limitations highlighted above, various experimental attempts have been made to obtain taxol from cellular taxus cultures, since in vitro cultures theoretically represent an unlimited source of the desired compound, thanks also to the possibility of using bioreactors that exert precise control on the production process.

It is worthwhile noting that the plant tissue culture is obtained from explants of differentiated tissue such as, for example, leaf, stem or root. The fragments obtained must be sterilised and then transferred, in aseptic conditions, in solid culture medium containing appropriate nutrients, vitamins, agar and phytohormones. These latter comprise two classes of compounds, auxins and cytokinins, often used in association with each other to promote a balanced cell growth and to induce the production of callus, i.e. a mass of tissue consisting of totipotent undifferentiated cells that are formed on the side of the explant two or three weeks after culturing. The callus can be propagated indefinitely, by transferring small fragments thereof into fresh medium, or else it can differentiate into root, leaf, bud, from which it is possible to induce the regeneration of a new plant. It is also possible to obtain cell cultures from the callus, upon transfer into liquid medium.

Cell cultures can produce secondary metabolites and release them into the culture medium or conserve them within the cells.

In the case of taxus, numerous authors have highlighted that cell cultures obtained from callus of different species of *Taxus* are capable of producing taxol, 10-deacetylbaccatin III and other taxanes (Wu et al., 2000, 2001; Cusidò et al., 1999; Shen et al., 1999).

Patent application EP 0 568 821 describes a method for inducing callus cells capable of producing taxanes from explanted tissues and refers to plant tissues of the family of the *Taxaceae.*

Other documents that describe the production of taxol and/or taxanes from cell and tissue cultures of the *Taxus* species are application WO 95/02063, patent US 5 019 054, application WO 92/13961 and application WO 95/02063.

Although taxol has always been considered a special product of the metabolism of the genus *Taxus,* several studies have highlighted that some species of fungi and bacteria *(Taxomices andreanae, Pestalotiopsis microspora*) that infest taxus are also capable of producing taxol and taxanes (Stierle et al., 1995; Pulici et al., 1997).

Hoffman and co-workers have shown that some American varieties of *Corylus avellana* (scientific name of hazel) contaminated by fungal pathogens have, in the leaves, in the branches and in the fruits, small amounts of taxol and other taxanes (Hoffman et al., 1998).

The amounts found are not, however, such as to realistically consider the possibility of extracting taxol and taxanes from the hazel plant.

Despite the numerous attempts to increase the production of taxol, its availability is still limited, due to the relative presence of taxol and taxanes in the differentiated taxus tissues and due to the difficulty in growing taxus cells *in vitro.*

In the light of the above it is clear that there is the requirement to find new sources of taxol and/or of its taxane precursors.

### Summary of the invention

The problem forming the basis of the present invention was therefore that of providing a method for the production of taxol and/or taxanes from an alternative source to the varieties of taxus used so farfor the direct extraction of taxol and taxanes or for their production through cell *cultures in vitro.*

Such a problem has been solved, according to the invention, by a method for the production of taxol and/or taxanes, comprising the steps of:
- inducing the formation of callus from a tissue explant of a plant capable of producing taxol and/or taxanes, through *in vitro* culturing in a suitable nutrient medium,
- cultivating said callus in a liquid medium to obtain a cell suspension culture capable of producing at least one taxane,-
- recovering said at least one taxane from the cells and/or from the culture medium of the cell suspension obtained, in which the aforementioned plant belongs to the genus *Corylus.*

Preferably, the aforementioned plant of the genus *Corylus* belongs to the species *Corylus avellana.*

The aforementioned at least one taxane is preferably selected from the group comprising taxol, 7-xylosyl-10-deacetyltaxol, 10-desacetylcephalomannine, 7-epitaxol, 10-desacetyl-7-epitaxol, 7-epicephalomannine, baccatin III, 10-desacetylbaccatin III, cephalomannine, taxagifine, 10-desacetyltaxol, xylosyl taxol, xylosyl cephalomannine, 7-epibaccatin III, 8-benzoyloxy taxagifine, 9-hydroxy taxusin, 9-acetyloxy taxusin, taxane Ia, taxane Ib, taxane Ic, taxane Id and taiwanxam.

The callus can be obtained from different parts of the plant, for example from leaves, stems, roots, seeds, etc., but it is preferably obtained from the seed.

In the induction step of the formation of the callus and in the subsequent callus cell culturing step in liquid suspension, one or more phytohormones can be added, preferably chosen from 2,4-dichlorophenoxyacetic acid (2,4-D), naphthaleneacetic acid (NAA), benzyladenine (BA) and mixtures thereof.

The use of a mixture of 2,4-D and BA in a weight ratio variable between 1:2 and 2:1, preferably at an overall concentration of between 0.5 and 4.0 mg/l and advantageously at a concentration of about 1.5 mg/l; or of a mixture of NAA and BA in a weight ratio variable between 2:1 and 5:1, preferably 4:1, preferably at an overall concentration of between 1.0 and 4.0 mg/l, advantageously about 2.5 mg/l or finally of hormone 2,4-D at a concentration of between 0.5 and 3.0 mg/l, advantageously between 0.5 and 1 mg/l, was particularly advantageous.

The nutrient medium used in the induction step of the formation of the callus preferably consists of a solid medium, where by solid medium a medium containing gelling agents is meant, for example agar, in sufficient quantity for the solidification of the medium.

The present invention refers, in a further aspect thereof, to the callus induced with the method described above and to the cells obtained from such a callus.

A considerable advantage of the method according to the present invention consists of the fact that it allows taxol and/or taxanes to be produced from a plant, hazel, which is widely available, which grows at a much faster rate than taxus and is substantially easier to cultivate or to regenerate *in vitro.*

Further characteristics and advantages of the method according to the present invention will become clearer from the following description of some preferred embodiments of the invention.

### Brief description of the drawings

Figure 1 is a chromatogram showing the separation profile of taxane standards by HPLC.
Figure 2a is a chromatogram of a taxol standard and figure 2b is the corresponding mass spectrum.
Figure 3a is a chromatogram of the taxol recovered in the supernatant of hazel cells at 15 days of cell culturing and figure 3b is the corresponding mass spectrum.
Figure 4a is a spectrum of the ions obtained from the MS/MS fragmentation of the standard taxol and figure 4b is the corresponding spectrum of the ions obtained from the MS/MS fragmentation of the taxol recovered in the supernatant of hazel cells at 15 days of culturing.

### Detailed description

The step of obtaining a tissue explant was carried out with different parts of *Corylus avellana* plants cultivated in Italy, in particular in Piedmont and in some areas of Liguria. The parts used were leaves, stems and seeds.

The explanted plant material was firstly sterilised by known methods, for example by immersion in a 5% NaClO solution for 20 minutes, and subsequently washed repeatedly with sterile distilled water.

The plant parts were then fragmented, working under a laminar flow sterile hood , and transferred into a solid culture medium.

Several conventional culture media can be used as solid culture medium, such as for example mB₅ medium (Gamborg B₅ medium), Durzan, MS (Murashige & Skoog), WPM (Lloyd & McCown), DKW (Driver-Kuniyuk-Walnut), LP (Quoirin & Lepiover), SH (Schenk & Hildebrandt) and Whites, preferably the MS medium (Murashige and Skoog 1962), with addition of a suitable amount of a gelling agent (e.g. agar at a concentration of about 10 g/l) and sucrose at a concentration of about 20 g/l.

The culture medium used contains one or more phytohormones at an overall concentration variable between 0.5 to 4.0 mg/l, with a preference for phytohormones of 2,4-dichlorophenoxyacetic acid (2,4-D), naphthaleneacetic acid (NAA), benzyladenine (BA) and mixtures thereof.

The fragments of explanted plant tissue are generally deposited in sterile Petri dishes containing one of the aforementioned solid media previously sterilised in an autoclave, generally at 121° for 15 minutes.

The dishes containing the plant material are then kept inside a phytochamber, in the dark and at a constant temperature comprised between 20 and 30°, preferably about 26°C.

After 15 days the fragments are transferred into new dishes with fresh culture medium and this transfer is repeated every 15 days, until the formation of a callus is obtained.

From the calluses obtained some fragments are selected for the preparation of the cell cultures. The selected fragments are transferred individually into a liquid culture medium, which can be one of those cited above, used for the induction of the callus, obviously without gelling agents or containing gelling agents in an amount such as not to cause the solidifying of the medium. Preferably, the MS medium is used, at the same concentration of phytohormones used for the induction of the callus.

The liquid culture medium can be contained in Erlenmeyer flasks or in other suitable containers, using medium suitable to promote gas exchanges and the dispersion of the cells (for example rotating dishes). The containers are kept at a constant temperature comprised between 20 and 30°C, preferably 26°C, and in the dark for at least 15 days.

To increase the production of taxol and/or taxanes, it may be advantageous to add elicitors, like for example vanadyl sulphate, 3,4-dichlorophenoxy triethylamine, methyljasmonate, chitosan ethylene and fungi like *Cytospora abietis, Penicillium miniluteum, Pestalotiopsis* (Ciddi et al., 1995; Mirjialili and Linden, 1996; Yukimune et al., 1996; Linden and Phisalaphong, 2000; Linden et al., 2001; Dong and Zhong, 2002; Yuang et al., 2002).

Periodically, the cell suspensions are centrifuged and the supernatant is separated from the pellet.

The taxol and/or taxanes produced can be extracted from the supernatant by known methods of the prior art, like for example the addition of adsorbing beads to the supernatant or the method that consists in filtering the supernatant with a 0.2-0.45 µm nylon filter (Linden et al., 2001), or with SPE columns and recovering taxanes with methanol.

The intracellular taxanes can be recovered by pulverising the pellet, dissolving it in methanol and filtering the solution obtained through a 0.2-0.45 µm nitrocellulose filter, to eliminate the cellular detritus.

The method according to the present invention will be further illustrated with reference to an example, provided hereafter for illustrating and non-limiting purposes.

### Example

The possibility of producing taxol and/or taxanes through hazel cell cultures was experimentally tested and compared with taxus cell cultures obtained in the same experimental conditions.

Therefore, *in vitro* cultures of plant tissues were prepared using leaves, stems and seeds of *Corylus avellana* (hazel) and of *Taxus baccata.* Both species used derive from land cultivations in Piedmont and Liguria.

All of the operations concerning the preparation and maintenance of the cultures *in vitro* were carried out under a vertical laminar flow sterile hood to ensure conditions of maximum sterility.

The plant material was first sterilised, immersing it in a 5% NaClO solution for 20 minutes, and then washed with sterile distilled water three times.

The plant parts were fragmented and transferred into sterile Petri dishes containing MS solid medium at a concentration of 4.3 g/l, containing vitamins (1 ml/l), sucrose (20 g/l), agar (10 g/l) and phytohormones at different concentrations, all previously sterilised in an autoclave at 121°C for 15 minutes.

In the preparation of cultures *in vitro* 2,4-dichlorophenoxyacetic acid (2,4-D), naphthaleneacetic acid (NAA), benzyladenine (BA) were used as phytohormones, in the following combinations and concentrations:
- 2,4-D 1 mg/l
- 2,4-D 0.5 mg/l
- BA 1 mg/l
- BA 0.5 mg/l
- 2,4-D 0.5 mg/l + BA 1 mg/l
- 2,4-D 1 mg/l + BA 0.5 mg/l
- NAA 2 mg/l
- NAA 2 mg/l + BA 0.5 mg/l

### Callus induction

For each type of plant tissue (leaf, stem, seed) and for each species (taxus, hazel) at least 96 dishes were prepared: 12 dishes for each hormone concentration orcombination. The dishes containing the plant material were kept inside a phytochamber, in the dark and at a constant temperature of 26°C.

The fragments were transferred, every 15 days, into new dishes with fresh medium containing the corresponding starting hormone or mixture of hormones. After 15 and 30 days from the culturing of the hazel differentiated tissue explants, the formation of the callus was evaluated. The formation of the callus for the taxus was evaluated at 30 and at 60 days, since before then the callus was not clear.

In table I and II the callus induction percentages for the species *Taxus baccata* and *Corylus avellana,* respectively, are shown, in relation to the different types of explants and the different hormone substrates.

**TABLE 1 callus induction from explants of Taxus baccata**

| HORMONE | CALLUS INDUCTION | | | | | |
|---|---|---|---|---|---|---|
| | 30 days | | | 60 days | | |
| | Leaves (%)* | Stems (%)* | Seeds (%)* | Leaves (%)* | Stems (%)* | Seeds (%)* |
| 2,4-D 1 mg/l | 25.3 | 67.0 | 26.5 | 56.3 | 76.3 | 37.7 |
| 2,4-D 0.5 mg/l | 10.9 | 46.8 | 17.4 | 63.8 | 65.8 | 43.7 |
| BA 1 mg/l | 2.0 | 2.5 | 0 | 3.0 | 2.8 | 0 |
| BA 0.5 mg/l | 2.0 | 2.0 | 6.2 | 4.5 | 9.0 | 32.7 |
| 2,4-D 0.5 mg/l + BA 1 mg/l | 4.0 | 16.0 | 8.2 | 8.0 | 24.3 | 21.0 |
| 2,4-D 1 mg/l + BA 0.5 mg/l | 22.3 | 22.3 | 2.5 | 30.3 | 32.5 | 16.0 |
| NAA 2 mg/l | 23.8 | 55.0 | 28.0 | 33.0 | 56.3 | 52.0 |
| NAA 2 mg/l + BA 0.5 mg/l | 0 | 9.5 | 5.0 | 10.0 | 12.0 | 13.0 |

| | | | | | | |
|---|---|---|---|---|---|---|
| * total number of explants / number of explants that produced callus. The reported value represents the average of at least three separate experiments for each type of explant. | | | | | | |

**TABLE II callus induction from explants of Corylus avellana**

| HORMONE | CALLUS INDUCTION | | | | | |
|---|---|---|---|---|---|---|
| | 15 days | | | 30 days | | |
| | Leaves (%)* | Stems (%)* | Seeds (%)* | Leaves (%)* | Stems (%)* | Seeds (%)* |
| 2,4-D 1 mg/l | 35.3 | 25.0 | 28.3 | 35.5 | 68.5 | 56.2 |
| 2,4-D 0.5 mg/l | 40.8 | 30.5 | 27.7 | 41.86 | 75.0 | 61.6 |
| BA 1 mg/l | 0 | 2.5 | 4.0 | 4.6 | 15.0 | 13.8 |
| BA 0.5 mg/l | 1.7 | 0 | 5.1 | 4.6 | 0 | 10.3 |
| 2,4-D 0.5 mg/l + BA 1 mg/l | 26.5 | 26.5 | 25.7 | 45.2 | 58.3 | 75.0 |
| 2,4-D 1 mg/l + BA 0.5 mg/l | 12.3 | 12.3 | 32.9 | 43.7 | 75.0 | 72.2 |
| NAA 2 mg/l | 8.3 | 8.3 | 4.4 | 22.1 | 5.4 | 41.3 |
| NAA 2 mg/l + BA 0.5 mg/l | 10.5 | 10.5 | 17.7 | 38.2 | 33.3 | 63.2 |

| | | | | | | |
|---|---|---|---|---|---|---|
| * total number of explants / number of explants that produced callus. The reported value represents the average of at least three separate experiments for each type of explant. | | | | | | |

After about three months from the formation of the callus, the best fragments (about 1-2 g) were selected for the preparation of cell cultures. In particular, for taxus cell cultures the callus grown both in dishes containing the 2,4-D hormone 0.5 mg/l, and in dishes with the NAA hormone 2 mg/l was picked out. For hazel cell cultures the callus induced with the following hormone concentrations was used:
- 2,4-D 0.5 mg/l
- 2,4-D 0.5 mg/l and BA 1 mg/l
- NAA 2 mg/l and BA 0.5 mg/l.

### Taxane extraction

The selected callus fragments were transferred individually into Erlenmeyer flasks containing 50 ml of liquid medium MS 4.3 g/l, vitamins 1 ml/l, saccharose 20 g/l and the same composition of phytohormones as the dish of origin. The flasks were placed on a rotating dish (125 revolutions/minute), to promote gas exchanges and the dispersion of the cells and kept at 26°C and in the dark for about three months. Every 15 days 25 ml of each cell culture were transferred into 25 ml of complete fresh medium, containing the relative hormones. The remaining 25 ml of the taxus and hazel cell cultures were centrifuged at 2500 revolutions/minute for 10 minutes and the cell pellet separated from the supernatant.

The supernatant and the pellet were treated separately for the extraction of the taxanes, following the method adopted by Linden (cited article).

To extract the extracellular taxanes, the supernatant was filtered with a 0.2-µm nylon filter (Gelman); the taxanes absorbed onto the membrane were recovered with 500 µl of methanol in a glass test tube for HPLC/MS analysis.

To extract the intracellular taxanes, the pellet was pulverised with liquid nitrogen and dissolved in 500 µl of methanol. To eliminate the cellular detritus, the solution obtained was filtered through a 0.2 µm nitrocellulose filter (Corning), and recovered for HPLC/MS analysis.

### HPLC / MS analysis

The chromatographic separation and the instrumental analysis were prepared by adjusting the chromatographic and instrumental detection parameters, using the commercial standards of the main taxanes of interest, i.e. 10-deacetylbaccatin III, Baccatin III, 10-deactetyl-7-xylosyltaxol, 10-deacetyltaxol and taxol.

For the chromatographic separation the HPLC Agilent 1100 instrument was used with a micro sampler and Agilent Zorbax C 18 column, having an inner diameter of 0.5 mm, length of 150 mm and particles with a diameter of 5 µm.

A mobile phase made up of acetonitrile and water, both containing 0.1% of trifluoroacetic acid, was used; the elution was carried out using a linear gradient: from 25% acetonitrile to 100% acetonitrile in 30 minutes, at a flow of 12 µl/min.

The injected sample volume was always of 8 µl.

The detector used was an Agilent 1100 ion trap mass spectrometer, equipped with electrospray source. The analysis was carried out by the positive ion scanning method.

The presence of taxanes in the hazel and taxus cell cultures was tested by comparing with the standards the following parameters: retention time, mass spectrum of the molecular ion, spectrum of the ions generated by MS/MS fragmentation.

The HPLC/MS analysis (confirmed with different methods such as ELISA), carried out every 15 days for a period of three months, on the cell cultures obtained from taxus and hazel callus, revealed the presence of taxanes both in the culture medium and in the cell pellet.

In particular, the cell cultures prepared with the method described above were capable of producing the taxanes corresponding to the standards used for the HPLC/MS analysis:
- 10-deacetylbaccatine III
- Baccatin III
- 10-deacetyl-7-xylosyltaxol
- 10-deacetyltaxol
- taxol

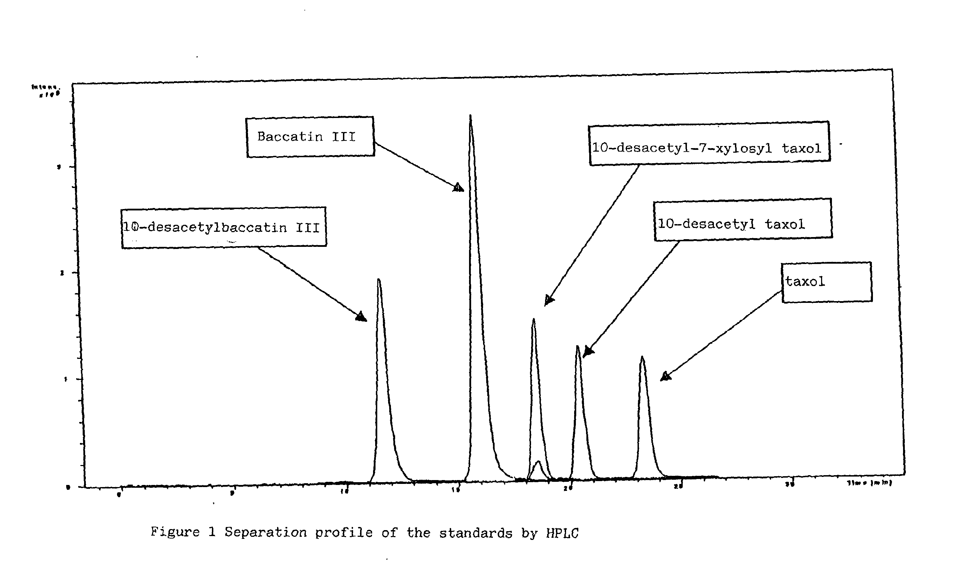

In fig. 1 is shown the separation profile of the standards obtained from the HPLC analysis.

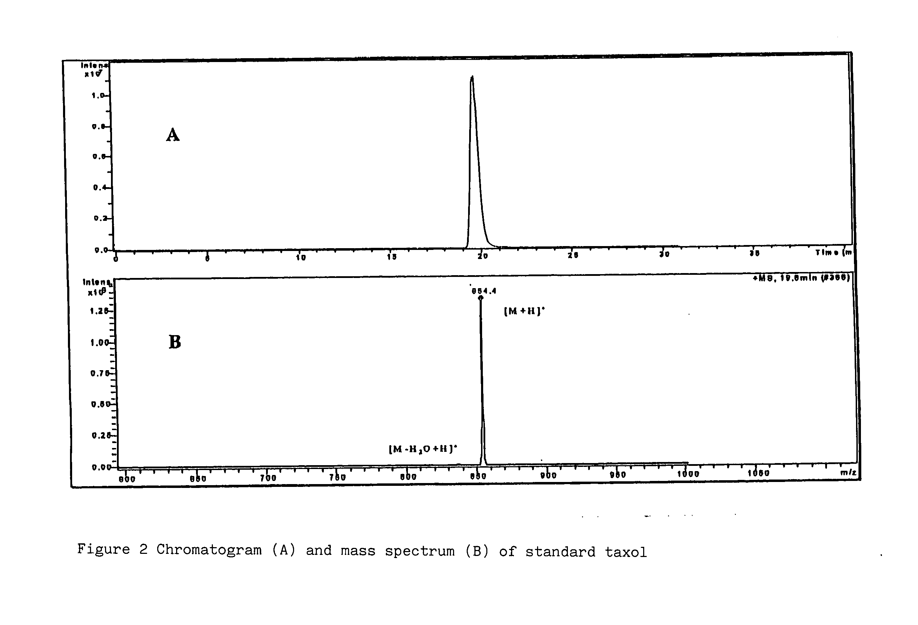

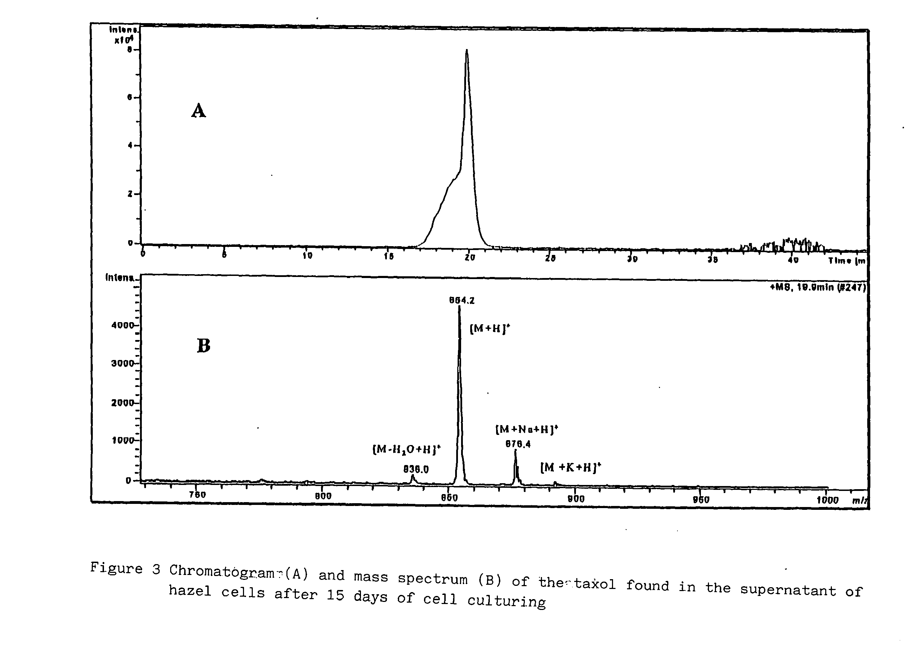

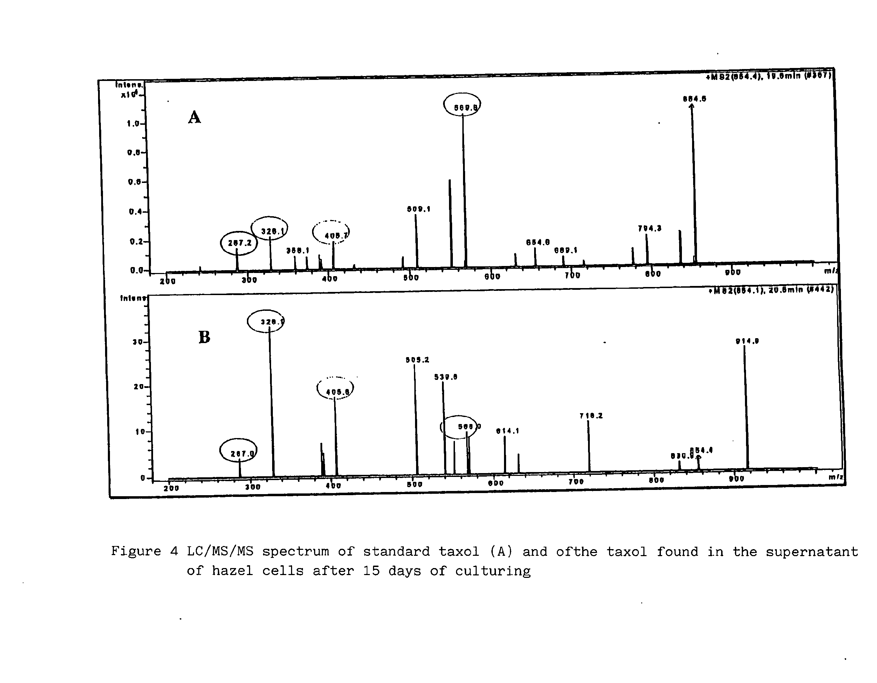

In figures 3 and 4 the data that indicate the recovery of the taxol in a sample of hazel cell cultures are shown, deducted from the precise correspondence of the analytical parameters of the sample with those of the corresponding standard. More precisely, in figures 3a and 3b the chromatogram and the mass spectrum of the taxol recovered from the supernatant of hazel cells at 15 days of cell culturing are shown. In figures 2a and 2b the chromatogram and the mass spectrum of the taxol used as standard are shown and in figures 4a and 4b the spectrum of the ions obtained from the MS/MS fragmentation of standard taxol and that of the taxol recovered in the hazel cell cultures are shown.

The presence of taxanes in the hazel has thus been confirmed and the production of taxol and taxanes in cell cultures of the same species has also been revealed for the first time. This suggests that also in the *C avellana* species there are enzymes involved in the metabolism of the taxanes.

It should also be noted that taxus and hazel are very different plants, both for growth and diffusion (the genus *Taxus* is somewhat rare and grows slowly, as opposed to the genus *Corylus,* which as well as being more common, also grows faster), and for genetic characteristics. Taxus, in fact, belongs to the group of the gymnosperms whereas hazel belongs to the group of the angiosperms. The first group comprises higher plants characterised by generally needle-shaped leaves and ovules that upon fertilisation transform into mainly woody strobili, whereas the second group comprises seed plants, endowed with flowers and ovules that, upon fertilisation, develop into fruits.

It is important to note that the *in vitro* cultivation of hazel, in particular starting from the seed, has proven to be more advantageous with respect to that of taxus; this is due to a greater ability of the hazel both of inducing callus from differentiated tissues of the plant (Table I), and of regenerating new young plants through the formation of buds from the undifferentiated callus tissue after about three months from the culturing of the different tissues, as shown in the data reported in the following table

**(Table III). Regeneration of callus of Taxus baccata and Corylus avellana**

| HORMONE | FORMATION OF YOUNG PLANTS (%)* | |
|---|---|---|
| | *T. baccata* | *C. avellana* |
| 2,4-D 1 mg/l | 0 | 15.5 |
| 2,4-D 0.5 mg/l | 0 | 15.8 |
| BA 1 mg/l | 0 | 8.7 |
| BA 0.5 mg/l | 0 | 13.3 |
| 2,4-D 0.5 mg/l + BA 1 mg/l | 0 | 15.0 |
| 2,4-D 1 mg/l + BA 0.5 mg/l | 0 | 17.1 |
| NAA 2 mg/l | 1 | 14.4 |
| NAA 2 mg/l + BA 0.5 mg/l | 0 | 16.2 |

| | | |
|---|---|---|
| *Total number of young plants / number of callus fragments analysed. | | |

The formation of callus from hazel was clear from 15 days after the culturing of the explants, whereas the taxus callus began to be assessable only after 30 days from the preparation of the dishes.

After 30 days of culturing the hazel reached the maximum callus induction percentage, even if with a large variability with respect to the hormone concentration and to the type of explant. The explants that have proven to be more productive were the seeds with a variable callus induction percentage of between 10.0% and 75%; the phytohormone that proved to be least suitable for inducing callus from hazel was BA when used alone at a concentration of 0.5 and 1 mg/l.

The taxus cultures reached the maximum callus induction after 60 days from culturing. Also in this case the BA phytohormone proved not to be very active, with the exception of the production of callus from seed revealed at 60 days for BA 1 mg/l.

Moreover, only one of the callus cultures obtained from taxus was capable of differentiating into bud and thus of producing a new young plant *in vitro* at a relatively low percentage (1%), whereas it was possible to obtain hazel buds and therefore young plants from callus induced in all of the hormone concentrations used by us with a greater frequency than that obtained for taxus.

Hazel therefore represents a rich and unlimited source of taxol and taxanes, both for its wide diffusion, and for the greater ease in being cultivated *in vitro* and in being regenerated in experimental conditions in which taxus regenerates with difficulty.

### References

Ciddi V, Srinivasan V, Shuler ML (1995). Elicitation of Taxus Sp. Cell cultures for production of Taxol. Biotechnology Letter, 17: 1343-6.
Cusidò RM, Palazon J, Navia-Osorio A, Mallol A, Bonfill M, Morales C, Pinol MT (1999). Production of taxol and baccatin III by a selected Taxus baccata callus line and its derived cell suspension culture. Plant Science, 146: 101-107.
Dong HD, Zhong JJ (2002): Enhanced taxane productivity in bioreactor cultivation of Taxus chinensis cells by combining elicitation, sucrose feeding and ethylene incorporation. Enzyme and Microb. Technol, 31: 116-121.
Hoffman A, Khan W, Worapong J, Strobel G, Griffin D, Arbogast B, Barotsky DF, Boone RB, Ning L, Zheng P, Daley LS (1998). Bioprospecting for Taxol in Angiosperm Plant Extracts. Spectroscopy, 13 (6): 22-32.
Holton RA, Biediger RJ, Boatman PD (1995). Semisynthesis of taxol and taxotere. In: Suffness M (ed) Taxol science and applications. CRC Press, Boca Raton, pp 97-121.
Holton RA, Kim HB, Somoza C, Liang F, Biediger RJ, Boatman PD, Vu P, Tang S, Zhang P, Murthi KK, Gentile LN, Liu JH (1994). First total synthesis of taxol 2. Completion of the C and D rings. J. Am. Chem. Soc, 116: 1599-1600.
Linden JC, Haigh JR, Mirjialili N, Phisalaphong M (2001). Gas concentration effects on secondary metabolite production by plant cell cultures. Advanced Biochemical Engineering/biotechnology, vol. 72 pp 28-61.
Linden JC, Phisalaphong M (2000). Oligosaccharides potentiate methyljasmonate-induced production of paclitaxel in Taxus Canadensis. Plant Science, 158:41-51.
Mc Guire TD, Schram KH, Reimer MLJ (1989). The mass spectrometry of taxol. J. Am. Soc. Mass Spectrom, 3: 672-679.
Mirjialili N and Linden JC (1996). Methyl Jasmonate Induced Production of taxol in suspension Cultures of Taxus cuspidate: Ethylene Interaction and Induction Models. Biotechnol. Prog, 12: 110-118.
Murashige T, Skoog F (1962). A revised medium for rapid growth and bioassay with tobacco tissue cultures. Plant Phisiol, 15: 473-97.
Pulici M, Sugawara F, Koshino H, Okada G, Esumi Y, Uzawa J, Yoshida S (1997). Metabolites of Pestalotiopsis spp, endophyticfungi of Taxus brevifolia. Phytochemistry, 46: 313-319.
Shen YC, Chen YJ, Chen CY (1999). Taxane diterpenoids from seeds of Chinese yew Taxus chinensis. Phytochemistry, 52: 1565-1569.
Shen YC, Chen YJ, Chen CY (1999). Taxane diterpenoids from seeds of Chinese yew Taxus chinensis. Phytochemistry, 52: 1565-1569.
Skeel RT (1999) Hanbook of chemiotherapy, 5 edn. Lippincott, Williams & Wilkins, Baltimore.
Stierle A, Strobel G, Stierle D, Grothaus P, Bignami G (1995). The search for a taxol-producing microorganism among the endophytic fungi of the pacific Yew, Taxus brevifolia. J Nat Prod, 58: 1315-1324.
Woo DDL, Miao SYP, Pelayo JC, Woolf AS (1994). Taxol inhibits progression of congenital polycistic kidney disease. Nature, 368: 750-753.
Wani MC, Taylor HL, Wall ME, Coggon P, Mc Phail AT (1971). Plant antitumor agents. VI. The isolation and structure of taxol. A novel antileukemic and antitumor agent from Taxus brevifolia. J Am Chem Soc, 93: 2325-2327.
Wu ZL, Yuan YJ, Ma ZH, Hu ZD (2000). Kinetics of two-liquid-phase Taxus cuspidate cell culture for production of taxol. Biochemical Engineering Journal, 5:147-142.
Wu J, Wang C, Mei X (2001). Stimulation of taxol production and excretion in Taxus spp cell cultures by rare earth chemical lanthanum. J Biotech, 85: 67-73.
Yuang YJ, Li C, Hu ZD, Wu JC, Zeng AP (2002). Fungal elicitor-induced apoptosis in suspension cultures of Taxus chinensis var. mairei for taxol production. Proc. biochem, 38: 193-198.
Yukimune Y, Tabata H, Higashi Y, Hara Y. (1996). Methyl jasmonate-induced overproduction of paclitaxel and baccatin III in Taxus cell suspension cultures. Nature biotechnology, 14: 1129-1130.

## Claims

1. Method for the production of taxol and/or taxanes, comprising the steps of:
a) inducing the formation of callus from a tissue explant of a plant capable of producing taxol and/or taxanes, through *in vitro* culture in a suitable nutrient medium,
b) cultivating said callus in a liquid medium to obtain a suspension cell culture capable of producing at least one taxane,
c) recovering said at least one taxane from the cells and/or from the medium of said cell culture, **characterized in that** said plant belongs to the genus *Corylus.*

2. Method according to claim 1, wherein said plant of the genus *Corylus* belongs to the species *Corylus avellana.*

3. Method according to claim 1 or 2, wherein said at least one taxane is selected from the group comprising taxol, 7-xylosyl-10-deacetyltaxol, 10-desacetylcephalomannine, 7-epitaxol, 10-desacetyl-7-epitaxol, 7-epicephalomannine, baccatin III, 10-desacetylbaccatin III, cephalomannine, taxagifine, 10-desacetyltaxol, xylosyl taxol, xylosyl cephalomannine, 7-epibaccatin III, 8-benzoyloxy taxagifine, 9-hydroxy taxusin, 9-acetyloxy taxusin, taxane Ia, taxane Ib, taxane Ic, taxane Id and taiwanxam.

4. Method according to any one of the previous claims, wherein said callus is obtained from the seed of said plant.

5. Method according to any one of the previous claims, wherein in said step a) of induction of the formation of the callus and in the subsequent one b) of culturing of the callus in liquid suspension, at least one phytohormone is added to said culture medium.

6. Method according to claim 5, wherein said at least one phytohormone is selected from 2,4-dichlorophenoxyacetic acid (2,4-D), naphthaleneacetic acid (NAA), benzyladenine (BA) and mixtures thereof.

7. Method according to claim 6, wherein said at least one phytohormone is selected from the group comprising 2,4-D at an overall concentration comprised between 0.5 and 3.0 mg/l, preferably between 0.5 and 1.0 mg/l, a 2,4-D/BA mixture in a weight ratio variable between 1:2 and 2:1 and an NAA/BA mixture in a weight ratio between 2:1 and 5:1, preferably 4:1.

8. Method according to claim 7, wherein said 2,4-D/BA mixture is present in the culture medium at an overall concentration comprised between 0.5 and 4.0 mg/l, preferably about 1.5 mg/l, and said NAA/BA mixture is present in the culture medium in an overall concentration comprised between 1.0 and 4.0 mg/l, preferably about 2.5 mg/l.

9. Method according to any one of the previous claims, wherein said nutrient medium used in the induction step of the formation of the callus consists of a solid medium.

10. Method according to any one of the previous claims, wherein at least one elicitor is added to the culture medium of said step b) selected from the group comprising vanadyl sulphate, 3,4-dichlorophenoxy triethylamine, methyl jasmonate, chitosan, ethylene and fungi such as *Cytospora abietis, Penicillium miniluteum, Pestalotiopsis.*

11. Callus obtained from plants of the genus *Corylus* through the method according to any one of the previous claims.

12. Callus cells obtained from plants of the genus *Corylus* through the method according to any one of claims 1 to 10.

13. Use of a part of a plant of the genus *Corylus* in the production of taxol and/or taxanes through *in vitro* cell culture.

14. Use according to claim 13, wherein said part consists of the seed.

15. Use according to claim 13 or 14, wherein said plant belongs to the species *Corylus avellana.*
